# FASCICULE DE BREVET EUROPEEN

(11) **EP 2 170 801 B1**
(45) Date de publication et mention de la délivrance du brevet: **26.03.2014**
(21) Numéro de dépôt: 08774645.9
(22) Date de dépôt: 02.07.2008
(51) Int. Cl.: C07C 67/11, C07C 69/712

(54) **NOUVEAU PROCEDE DE SYNTHESE DU FENOFIBRATE**
NEUES VERFAHREN ZUR SYNTHESE VON FENOFIBRAT
NOVEL METHOD OF SYNTHESIZING FENOFIBRATE

(30) Priorité: 02.07.2007 FR 0756220
(43) Date de publication de la demande: 07.04.2010
(73) Titulaire: Synkem, 21300 Chenove (FR)
(72) Inventeur: LUCAS, Béatrice, 21490 Brognon (FR)
(74) Mandataire: Hubert, Philippe
(86) Numéro de dépôt international: PCT/EP2008/058511
(87) Numéro de publication internationale: WO 2009/004029

(56) Documents cités:
- EP-A- 0 126 361
- WO-A-02/062743
- FR-A- 2 300 552
- L.BOROS ET AL.: "Preparation of new 2,3-diphenylpropenoic acid esters- good yields even for the more hindered Z isomers" MOLECULES, vol. 9, 2004, pages 256-263, XP009096140 cité dans la demande

## Description

L'invention concerne un nouveau procédé de synthèse du fénofibrate.

### Art antérieur

Le fénofibrate (méthyléthyl ester de l'acide 2-[4-(4-chlorobenzoyl)-phénoxy]-2-méthylpropanoique) 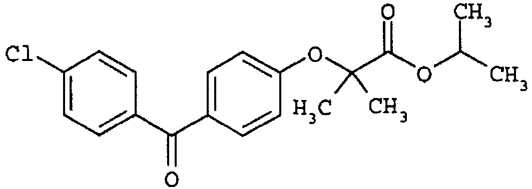 est un principe actif connu pour traiter les hypertriglycéridémies et les hypercholestérolémies.

Ce composé est un ester isopropylique de l'acide fénofibrique et différents procédés ont été proposés pour sa fabrication industrielle.

Ainsi, le fénofibrate est préférentiellement obtenu avec un bon rendement par réaction de la 4-chloro-4'-hydroxybenzophénone avec le 2-bromo-2-méthylpropanoate d'isopropyle, en l'absence de solvant et en présence d'un agent alcalin tel que notamment le carbonate de potassium (EP0245156B1).

Le document WO 02/062743 divulgue un procédé de préparation de fibrates voisin du procédé précédent, mais dans lequel le phénol est mis en réaction avec un 2-bromo-2-méthyl propionate d'alkyle en présence de bicarbonate de potassium et dans un solvant choisi parmi les alcools et les cétones en C1-C4. Le seul exemple décrit dans ce document est relatif au fénofibrate et le solvant utilisé est l'isopropanol . Le temps de réaction est cependant beaucoup plus important que dans le cas du procédé sans solvant.

Le document EP0002151 divulgue un procédé différent selon lequel on fait réagir l'ester méthylique de l'acide 2-méthyl-2-phénoxypropanoïque avec le chlorure de l'acide 4-chlorobenzoïque ou l'anhydride 4-chlorobenzoïque dans un solvant halogéné en présence d'un acide de Lewis tel que le trifluorure de bore (réaction de Friedel-Craft). Ce procédé ne donne cependant pas de résultats satisfaisants si l'on utilise l'ester isopropylique qui permettrait d'obtenir directement le fénofibrate et la fabrication du fénofibrate nécessite alors une transestérification en présence d'isopropylate de sodium.

On connaît encore les procédés décrits dans les demandes de brevet FR 2035821, FR 2157853 (certificat d'addition au brevet français FR 2035821), FR 2300552 et FR 2342723. Parmi ces derniers procédés, une méthode de synthèse industrielle consiste en :
a) préparation de la 4-chloro-4'-hydroxybenzophénone par réaction de Friedel-Craft entre le chlorure de 4-chlorobenzoyle et l'anisole, puis déméthylation du composé obtenu,
b) préparation de l'acide 2-[4-(4-chlorobenzoyl)phénoxy]-2-méthylpropanoique) par réaction du mélange acétone-chloroforme en milieu alcalin (réaction dite de Bargellini) avec la 4-chloro-4'-hydroxybenzophénone,
c) préparation du fénofibrate par estérification de l'acide avec l'alcool isopropylique en milieu acide, par exemple en présence d'acide sulfurique.

Cette dernière méthode présente l'avantage d'utiliser des réactifs peu coûteux et donc d'obtenir le fénofibrate avec un coût matières intéressant. Cependant, la mise en oeuvre industrielle de l'étape d'estérification nécessite des quantités importantes d'acide sulfurique pour obtenir une bonne cinétique de réaction et il s'en suit de fréquentes difficultés de mise en oeuvre.

On connaît par ailleurs, de la littérature, des méthodes de synthèse pour obtenir un ester au départ d'un acide carboxylique faisant appel à la réaction d'un halogénure d'alkyle avec un sel métallique dudit acide carboxylique. Par exemple la publication Molecules 2004, 9, 256-263 propose la réaction de l'acide E- ou Z-2,3-diphénylpropènoïque avec le bromure d'isopropyle afin d'obtenir l'ester isopropylique correspondant. Les rendements sont cependant faibles, de l'ordre de 36 à 38 %, ce qui n'est pas compatible avec une production industrielle.

Le brevet US 3,649,655 décrit la préparation de l'acétate de cyclohexyle par réaction du bromure de cyclohexyle avec l'acide acétique en présence d'un catalyseur minéral tel que le chlorure ferrique. Dans ce cas également, les rendements de réaction ne dépassent pas 66%.

Plus récemment, la publication Tetrahedron Letters 46 (2005) 3641-3644 divulgue de bons rendements -de l'ordre de 85 à 94%- pour la préparation d'esters isopropyliques au départ de 2-bromopropane et d'acides benzoïques diversement substitués. La réaction est cependant conduite dans un solvant liquide ionique [(trihexyl)tétradécyl-phosphonium bis-(trifluorométhyl-sulfonyl)-amide] dont l'utilisation au stade industriel ne paraît pas envisageable dans l'immédiat.

### Objet de l'invention

On a maintenant découvert, et ceci constitue le fondement de l'invention, qu'il était possible de réaliser l'estérification de l'acide fénofibrique avec un halogénure d'isopropyle afin d'obtenir le fénofibrate, dans des conditions économiques tout à fait compatibles avec une fabrication à l'échelle industrielle, c'est-à-dire la production de plusieurs centaines de tonnes chaque année, par le choix d'un solvant réactionnel spécifique.

Plus précisément, il a été découvert, d'une façon tout à fait inattendue, que l'utilisation d'un solvant constitué d'un mélange en toute proportion de diméthylsulfoxyde (DMSO) et d'un acétate d'alkyle en C₂-C₄ permet un accès direct à un fénofibrate de qualité conforme à la pharmacopée, sans nécessiter une purification complémentaire par recristallisation.

Le procédé de préparation du fénofibrate selon l'invention est ainsi caractérisé en ce qu'il consiste à faire réagir, dans un solvant constitué d'un mélange de diméthylsulfoxyde et d'un acétate d'alkyle en C₂-C₄, un sel métallique de l'acide fénofibrique avec un halogénure d'isopropyle.

En d'autres termes, la présente invention concerne un nouveau procédé d'obtention du fénofibrate au départ de l'acide fénofibrique, dans lequel la réaction d'estérification est réalisée à l'aide d'un halogénure d'alkyle et conduite dans un solvant spécifiquement choisi, selon le schéma réactionnel :

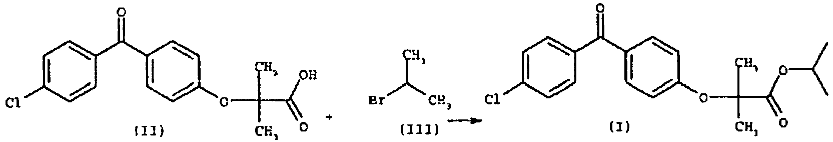

Un mode général de réalisation de l'invention consiste à faire réagir l'acide fénofibrique avec une base minérale, dans un système solvant constitué d'un mélange de diméthylsulfoxyde et d'un acétate d'alkyle en C₂-C₄, de façon à obtenir un sel métallique de l'acide fénofibrique tel que par exemple un sel de potassium ou de sodium, puis à faire réagir ce sel avec un halogénure d'isopropyle, à une température proche de la température de reflux du mélange réactionnel à la pression atmosphérique, pour obtenir le fénofibrate en solution dans le mélange.

L'originalité de la présente invention réside dans le choix du système solvant utilisé pour la mise en oeuvre de la réaction représentée par le schéma réactionnel qui précède.

Il a été découvert que l'association du diméthylsulfoxyde et d'un acétate d'alkyle en C₂-C₄ permet de conduire la réaction précitée dans les meilleures conditions et d'obtenir directement un produit de qualité conforme à la pharmacopée ne contenant aucune impureté à un taux supérieur à 0,05 %.

Dans ce contexte, il a été montré :
- d'une part, que le diméthylsulfoxyde (DMSO) autorise à la fois un bon rendement chimique et une bonne qualité du fénofibrate obtenu, et
- d'autre part, que l'utilisation conjointe du DMSO et d'un acétate d'alkyle en C₂-C₄, de préférence l'acétate d'isopropyle, permet de conduire la réaction dans les meilleures conditions et d'obtenir directement un produit de qualité conforme à la pharmacopée.

Les quantités de diméthylsulfoxyde et d'acétate d'alkyle en C₂-C₄ utilisées dans le cadre de la présente invention peuvent varier dans de larges limites.

D'excellents résultats ont été obtenus en conduisant la réaction avec une quantité de diméthylsulfoxyde telle que le rapport DMSO/acide fénofibrique (exprimé en poids) est compris entre 0,1 et 2, et préférentiellement entre 0,2 et 1.

Selon un mode de réalisation actuellement préféré, on utilisera un mélange de DMSO et d'acétate d'alkyle tel que le rapport entre le poids total de solvant et le poids de l'acide fénofibrique soit compris entre 0,2 et 3, et plus préférentiellement entre 0,4 et 2.

L'halogénure d'isopropyle est de préférence le bromure d'isopropyle (encore dénommé 2-bromopropane).

Le sel métallique de l'acide fénofibrique est généralement préparé par réaction (conduite de préférence dans le milieu réactionnel) entre l'acide fénofibrique et une base, de préférence une base minérale.

Parmi les bases minérales susceptibles d'être utilisées pour préparer le sel métallique d'acide fénofibrique, on peut citer les hydroxydes, carbonates ou bicarbonates de potassium ou de sodium, ainsi que les hydroxydes ou carbonates de lithium ou calcium.

D'excellents résultats ont été obtenus à l'aide du carbonate de potassium qui constitue donc une base minérale particulièrement préférée.

Selon un mode de réalisation préféré de l'invention, on salifie l'acide fénofibrique avec du carbonate de potassium et on fait réagir le sel de potassium obtenu avec le bromure d'isopropyle au sein du même milieu réactionnel.

### Description détaillée de l'invention :

Selon un mode opératoire général du procédé selon l'invention, on prépare d'abord un mélange constitué d'acide fénofibrique et du système solvant auquel on ajoute une quantité stoechiométrique d'un composant basique minéral susceptible de former un sel avec l'acide fénofibrique. On utilise plus particulièrement le carbonate de potassium pour obtenir le sel de potassium de l'acide fénofibrique. On ajoute ensuite au milieu réactionnel une quantité légèrement supérieure à la stoechiométrie d'un halogénure d'isopropyle (de préférence de 2-bromopropane) et on porte le mélange réactionnel à doux reflux, pendant 2 à 8 heures. Les sel minéraux insolubles sont éliminés par filtration et le solvant de la réaction est éliminé et remplacé par le solvant de cristallisation du fénofibrate. Après refroidissement le fénofibrate cristallisé est isolé par filtration sur essoreuse et séché.

L'exemple suivant, qui décrit un mode opératoire réalisé au stade laboratoire, permet de mieux comprendre l'invention. Cet exemple ne doit cependant pas être considéré comme limitatif et le réactif de salification, l'halogénure d'isopropyle et les solvants peuvent être modifiés sans sortir du cadre de l'invention.

### EXEMPLE I

### Acide 2-[4-(4-chlorobenzoyl)phénoxy]-2-méthyl-propanoïque, 1-méthyléthyl ester

Dans un réacteur double enveloppe de 5 1 inerté à l'azote, on a chargé 1 kg (3,14 moles) d'acide fénofibrique (II), 500 ml de diméthylsulfoxyde et 1 1 d'acétate d'isopropyle. Puis sous agitation et à température ambiante, on a ajouté 433,5 g (3,14 moles) de carbonate de potassium et on a porté le mélange réactionnel à 85-90°C pendant 45 mn. La température du milieu a ensuite été descendue vers 80°C et on a ajouté, en 50 mn, 354 ml (3,77 moles) de 2-bromopropane, puis 100 ml d'acétate d'isopropyle. Le mélange a été maintenu sous agitation à 85-95°C pendant 5 heures puis refroidi légèrement vers 80°C. Un contrôle en cours de procédé a montré que le taux de conversion en fénofibrate était d'environ 99,5%. Le contenu du réacteur a été filtré à chaud et les sels séparés sur le filtre ont été lavés avec 1 l d'acétate d'isopropyle que l'on a joint au filtrat. Les sels isolés, qui contenaient essentiellement du bromure de potassium, ont été séchés et récupérés pour recyclage. Le filtrat a été concentré sous pression réduite tout en maintenant une température dans la masse proche de 80°C, puis de 95°C en fin de concentration. Après élimination des solvants, on a ajouté 2,27 l d'isopropanol et 455 ml d'eau pure. Le mélange a été porté à doux reflux du solvant pendant 10 mn puis filtré à chaud. Le filtrat a été refroidi doucement sous agitation jusqu'à une température de 0°C. Le fénofibrate cristallisé a été séparé par filtration sur essoreuse, lavé sur l'essoreuse par environ 500 ml d'isopropanol glacé, puis séché sous vide à 45-50°C. On a ainsi obtenu 1075 g de fénofibrate de pureté supérieure à 99,5% et ne contenant aucune impureté à un taux supérieur à 0,05% (rendement = 94,9%).

Ce procédé est transposable en réacteur industriel (acier inoxydable ou vitrifié) et permet la production de lots de 1000 Kg environ avec des réacteurs de 40001, dans des conditions de capacités et de temps réactionnels compatibles avec une excellente productivité.

Le procédé selon l'invention permet un accès direct à un produit de qualité conforme à la pharmacopée sans la nécessité d'une purification par recristallisation. Ces différents aspects sont également très intéressants du point de vue de la protection de l'environnement puisque les sous produits de la réaction sont en quantité limitée et pour la plupart recyclables. Sur ce point particulier, il est également remarquable que l'ensemble du procédé utilise très peu d'eau et ne produit pas de déchets sous forme de solutions aqueuses salines.

## Revendications

1. Procédé de préparation du fénofibrate, **caractérisé en ce que** l'on fait réagir, dans un solvant constitué d'un mélange de diméthylsulfoxyde et d'un acétate d'alkyle en C₂-C₄, un sel métallique de l'acide fénofibrique avec un halogénure d'isopropyle.

2. Procédé selon la revendication 1, **caractérisé en ce que** le solvant précité est un mélange de diméthylsulfoxyde et d'acétate d'isopropyle.

3. Procédé selon la revendication 1 ou 2, **caractérisé en ce que** le rapport diméthylsulfoxyde/acide fénofibrique (exprimé en poids) est compris entre 0,1 et 2.

4. Procédé selon la revendication 1 ou 2, **caractérisé en ce que** le rapport diméthylsulfoxyde/acide fénofibrique (exprimé en poids) est compris entre 0,2 et 1

5. Procédé selon l'une des revendications 1 à 4, **caractérisé en ce que** le solvant précité est utilisé en une quantité telle que le rapport entre le poids total du solvant et le poids de l'acide fénofibrique est compris entre 0,2 et 3.

6. Procédé selon la revendication 5, **caractérisé en ce que** le rapport entre le poids total de solvant et le poids de l'acide fénofibrique est compris entre 0,4 et 2.

7. Procédé selon l'une des revendications 1 à 6, **caractérisé en ce que** l'halogénure d'isopropyle est le bromure d'isopropyle (ou 2-bromopropane).

8. Procédé selon l'une quelconque des revendications 1 à 7, **caractérisé en ce que** le sel métallique de l'acide fénofibrique est choisi parmi les sels de potassium, de sodium de lithium ou de calcium.

9. Procédé selon la revendication 8, **caractérisé en ce que** le sel métallique de l'acide fénofibrique est le sel de potassium.

## Patentansprüche

1. Verfahren zur Herstellung von Fenofibrat, **dadurch gekennzeichnet, dass** man in einem Lösungsmittel, das aus einem Gemisch aus Dimethylsulfoxid und einem C₂-C₄-Alkylacetat besteht, ein Metallsalz der Fenofibrinsäure mit einem Isopropylhalogenid reagieren lässt.

2. Verfahren nach Anspruch 1, **dadurch gekennzeichnet, dass** das vorgenannte Lösungsmittel ein Gemisch aus Dimethylsulfoxid und Isopropylacetat ist.

3. Verfahren nach Anspruch 1 oder 2, **dadurch gekennzeichnet, dass** das Verhältnis Dimethylsulfoxid / Fenofibrinsäure (ausgedrückt in Gewicht) zwischen 0,1 und 2 beträgt.

4. Verfahren nach Anspruch 1 oder 2, **dadurch gekennzeichnet, dass** das Verhältnis Dimethylsulfoxid / Fenofibrinsäure (ausgedrückt in Gewicht) zwischen 0,2 und 1 beträgt.

5. Verfahren nach einem der Ansprüche 1 bis 4, **dadurch gekennzeichnet, dass** das vorgenannte Lösungsmittel in einer solchen Menge verwendet wird, dass das Verhältnis zwischen dem Gesamtgewicht des Lösungsmittels und dem Gewicht der Fenofibrinsäure zwischen 0,2 und 3 beträgt.

6. Verfahren nach Anspruch 5, **dadurch gekennzeichnet, dass** das Verhältnis zwischen dem Gesamtgewicht an Lösungsmittel und dem Gewicht der Fenofibrinsäure zwischen 0,4 und 2 beträgt.

7. Verfahren nach einem der Ansprüche 1 bis 6, **dadurch gekennzeichnet, dass** das Isopropylhalogenid Isopropylbromid (oder 2-Brompropan) ist.

8. Verfahren nach einem der Ansprüche 1 bis 7, **dadurch gekennzeichnet, dass** das Metallsalz der Fenofibrinsäure aus den Kalium-, Natrium-, Lithium- oder Kalziumsalzen ausgewählt ist.

9. Verfahren nach Anspruch 8, **dadurch gekennzeichnet, dass** das Metallsalz der Fenofibrinsäure Kaliumsalz ist.

## Claims

1. A method for the preparation of fenofibrate, **characterized in that** the reaction of a metal salt of fenofibric acid with an isopropyl halide is carried out in a solvent composed of a mixture of dimethyl sulfoxide and of a C₂-C₄ alkyl acetate.

2. The method as claimed in claim 1, **characterized in that** said solvent is a mixture of dimethyl sulfoxide and of isopropyl acetate.

3. The method as claimed in claim 1 or 2, **characterized in that** the dimethyl sulfoxide/fenofibric acid ratio (expressed by weight) is comprised between 0.1 and 2.

4. The method as claimed in claim 1 or 2, **characterized in that** the dimethyl sulfoxide/fenofibric acid ratio (expressed by weight) is comprised between 0.2 and 1.

5. The method as claimed in one of claims 1 to 4, **characterized in that** said solvent is used in an amount such that the ratio of the total weight of solvent to the weight of fenofibric acid is comprised between 0.2 and 3.

6. The method as claimed in claim 5, **characterized in that** the ratio of the total weight of solvent to the weight of fenofibric acid is comprised between 0.4 and 2.

7. The method as claimed in one of claims 1 to 6, **characterized in that** the isopropyl halide is isopropyl bromide (or 2-bromopropane).

8. The method as claimed in any one of claims 1 to 7, **characterized in that** the metal salt of fenofibric acid is chosen from a potassium, sodium, lithium or calcium salts.

9. The method as claimed in claim 8, **characterized in that** the metal salt of fenofibric acid is a potassium salt.
